# EUROPEAN PATENT SPECIFICATION

(11) **EP 2 033 568 B1**
(45) Date of publication and mention of the grant of the patent: **23.11.2011**
(21) Application number: 07767594.0
(22) Date of filing: 26.06.2007
(51) Int. Cl.: A61B 1/00, A61B 1/06, A61B 5/07

(54) **BODY INSERTABLE APPARATUS**
IN DEN KÖRPER EINFÜHRBARES GERÄT
DISPOSITIF INSERABLE DANS LE CORPS

(30) Priority: 26.06.2006 JP 2006175559
(43) Date of publication of application: 11.03.2009
(73) Proprietor: Olympus Medical Systems Corp., Tokyo 151-0072 (JP)
(72) Inventor: KAGAWA, Ryohei, Shibuya-ku, Tokyo 151-0072 (JP)
(74) Representative: von Hellfeld, Axel
(86) International application number: PCT/JP2007/062788
(87) International publication number: WO 2008/001763

(56) References cited:
- WO-A-97/33513
- JP-A- 2004 328 941
- JP-A- 2005 143 670
- US-A1- 2003 020 810
- US-A1- 2004 215 084

## Description

### TECHNICAL FIELD

The present invention relates to a body-insertable apparatus such as a capsule endoscope which performs various types of medical practices in a body of a subject, including an examination and a treatment in a body cavity.

### BACKGROUND ART

In recent years, a swallowing capsule endoscope as a body-insertable apparatus to be inserted into a body of a subject such as a patient has appeared in the field of an endoscope. The capsule endoscope is provided with an imaging function and a radio communication function. The capsule endoscope has functions of traveling, after it is swallowed from a mouth of a patient for the purpose of an observation (examination) and until it is naturally excreted from a human body, an inside of a body cavity, for example, an inside of organs such as the stomach and the small intestine according to their peristalsis, and of sequentially capturing their images.

Image data captured in the body by the capsule endoscope, while the capsule endoscope travels the inside of the body cavity, is sequentially transmitted to an outside through a radio communication, and stored in a memory provided in an external receiver. The patient carrying the receiver provided with the radio communication function and the memory function can move freely even during the period which starts when the capsule endoscope is swallowed and ends when the capsule endoscope is excreted. Thereafter, doctors or nurses make a display device display images of organs based on the image data stored in the memory to make a diagnosis (see Patent Documents 1 and 2).

Patent Document 1: Japanese Patent Application Laid-Open No. 2002-204781
Patent Document 2: Japanese Patent Application Laid-Open No. 2005-143670

WO 1997/33513 discloses a biosensing transponder for implantation in an organism, wherein power supplied to illumination means is saved by operating the illumination means in a pulsed manner, i.e., only energizing LEDs when a measurement is desired. A storage capacitor is used to provide pulse of electrical energy to the illumination means at power levels that are greater than the continuous power received by the energy coupler. By storing energy received from the control circuit in the capacitor, the illumination means can be indirectly energized by the control circuit through a pulsing discharge of the capacitor.

US 2003/020810 discloses a capsule endoscope according to the preamble of claim 1 having a capacitor disposed therein which stores charges from a battery to be used for cauterizing tissue through bipolar electrodes.

### DISCLOSURE OF INVENTION

### PROBLEM TO BE SOLVED BY THE INVENTION

However, since the conventional capsule endoscope described above uses a small battery as a power source in response to a necessity of downsizing the capsule endoscope itself, there are problems that a power capacity is limited and thereby a high power cannot be supplied at once. For this reason, there is a case where a clear image cannot be obtained with a limit to an amount of light emitted by an LED in performing the imaging.

In contrast, when the capacity or the number of the battery is increased to overcome the power limit, there are problems that a volume of the capsule endoscope itself is increased, which leads to a growth in size of the capsule endoscope itself, and that a volume to contain various function executing units including an imaging unit becomes small in a limited space in a chassis of the capsule endoscope, which leads to a difficulty in realizing desired functions sufficiently.

The present invention has been achieved in view of the foregoing, an object of the present invention is to provide a body-insertable apparatus which can instantly obtain high power while maintaining a small size with a small power source capacity.

### MEANS FOR SOLVING PROBLEM

A body-insertable apparatus according to claim 1 is provided. The body insertable apparatus, according to an aspect of the present invention includes a plurality of function executing units each of which executes a predetermined function and a power source which supplies an electric power to each of the function executing units. The body-insertable apparatus includes an electric power storing unit that is connected to the power source and temporarily stores a redundancy of the electric power supplied from the power source; and an electric power discharging switch that supplies the electric power stored during a temporal period by the electric power storing unit to a high-power function executing unit as a function executing unit that operates intermittently among the function executing units and temporarily consumes a large electric quantity compared to the other function executing units, wherein the high-power function executing unit consumes the electric power supplied from the electric power storing unit together with the electric power directly supplied from the power source.

In the body-insertable apparatus, the high-power function executing unit may include a driving switch which operates intermittently, and the electric power discharging switch may be turned on/off in synchronization with turning on/off of the driving switch.

The body-insertable apparatus may further include an electric power charging switch which is provided between the power source and the electric power storing unit, and switches on/off a power supply from the power source to the electric power storing unit.

In the body-insertable apparatus, the plurality of function executing units may include an illuminating unit that illuminates an imaging target in imaging; an imaging unit that captures an image of the imaging target illuminated by the illuminating unit; a radio communication unit that wirelessly transmits the image captured by the imaging unit; and a control unit that controls the illuminating unit, the imaging unit, and the radio communication unit.

In the body-insertable apparatus the high-power function executing unit may be the illuminating unit that illuminates the imaging target in imaging.

In the body-insertable apparatus, the high-power function executing unit may be a compressing unit that performs a compression processing on the image captured by the imaging unit.

In the body-insertable apparatus, the high-power function executing unit may be a signal processing circuit that transforms analogue image data captured by the imaging unit into digital image data.

In the body-insertable apparatus, the illuminating unit may include a plurality of LEDs constituted by an LED for emitting a light based on the electric power directly supplied from the power source and an LED for emitting a light based on the electric power supplied from the electric power storing unit.

In the body-insertable apparatus, the electric power storing unit may be a capacitor.

In the body-insertable apparatus, the power source may be a battery.

A body-insertable apparatus, according to another aspect of the present invention includes a plurality of function executing units each of which executes a predetermined function and a power source that supplies an electric power to each of the function executing units. The body-insertable apparatus includes an electric power storing unit that is connected to the power source and temporarily stores a redundancy of the electric power supplied from the power source; and an electric power discharging switch that supplies the electric power stored by the electric power storing unit to a high-power function executing unit as a function executing unit that, among the function executing units, consumes an electric quantity exceeding a threshold value arbitrarily set. The high-power function executing unit consumes the electric power supplied from the electric power storing unit together with the electric power directly supplied from the power source.

A body-insertable apparatus according to still another aspect of the present invention includes a plurality of function executing units each of which executes a predetermined function and a power source that supplies an electric power to each of the function executing units. The body-insertable apparatus includes an electric power storing unit that is connected to the power source and temporarily stores a redundancy of the electric power supplied from the power source; and an electric power discharging switch that supplies the electric power stored by the electric power storing unit to a high-power function executing unit as a function executing unit that, among the function executing units, consumes an electric quantity not less than an average electric power value. The high-power function executing unit consumes the electric power supplied from the electric power storing unit together with the electric power directly supplied from the power source.

### EFFECT OF THE INVENTION

A body-insertable apparatus according to the present invention has an advantageous effect that a high power can be obtained instantly while a small size is maintained with a small power source capacity since an electric power storing unit connected to a power source temporarily stores a redundancy of an electric power supplied from the power source; an electric power discharging switch supplies the electric power stored during the temporal period by the electric power storing unit to a high-power function executing unit as a function executing unit which operates intermittently among function executing units and temporarily consumes a large power compared to the other function executing units; and the high-power function executing unit consumes the electric power supplied from the electric power storing unit together with the electric power directly supplied from the power source.

### BRIEF DESCRIPTION OF DRAWINGS

FIG. 1 shows a schematic configuration of an intra-subject medical system including a capsule endoscope according to a first embodiment of the present invention.
FIG. 2 is a cross sectional view of the configuration of the capsule endoscope shown in FIG. 1.
FIG. 3 is a block diagram of the configuration of the capsule endoscope shown in FIG. 1.
FIG. 4 is a time chart showing a state of a power usage of the capsule endoscope shown in FIG. 1.
FIG. 5 is a block diagram of a configuration of a capsule endoscope according to a modification of the first embodiment of the present invention.
FIG. 6 is a time chart showing a state of a power usage of the capsule endoscope shown in FIG. 5.
FIG. 7 is a block diagram of a configuration of a capsule endoscope according to a second embodiment of the present invention.
FIG. 8 is a view showing an LED arrangement, seen from a forefront side where LED is arranged, in the capsule endoscope shown in FIG. 7.

### EXPLANATIONS OF LETTERS OR NUMERALS

- 1: Subject
- 2: Receiving device
- 2a: Antenna unit
- 2b: Receiving main body unit
- 3: Capsule endoscope
- 4: Display device
- 5: Portable recording medium
- 10: Illuminating unit
- 11,: 11a, 11b LED (LED group)
- 12,: 12a, 12b LED driver
- 16: Capsule-shaped casing
- 20: Power source
- 21: Battery
- 22: Power source circuit
- 23: Electric power storing unit
- 24: Electric power discharging switch
- 25: Driving switch
- 26: Electric power charging switch
- 30: Imaging unit
- 32: CCD
- 33: Imaging lens
- 40: Radio unit
- 41: Antenna
- 50: Signal processor/controller
- 61: Imaging substrate
- 62: Illuminating substrate
- 63: Power source circuit substrate
- 64, 65: Radio substrate
- 68: Flexible substrate

### BEST MODE(S) FOR CARRYING OUT THE INVENTION

A body-insertable apparatus as best mode(s) for carrying out the invention will be explained below with reference to the accompanying drawings. It should be noted that though exemplary embodiments of the present invention will be explained below by taking, as one example, a capsule endoscope which exemplifies the body-insertable apparatus to be inserted into a body of a subject such as a patient, the present invention is not limited to the specific embodiments.

### First embodiment

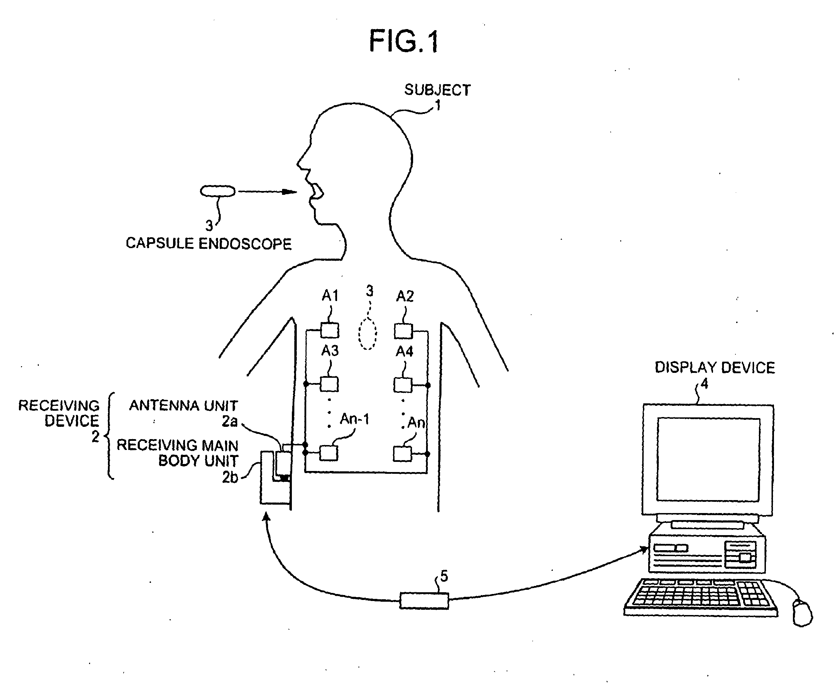

FIG. 1 is a schematic view of an entire configuration of an intra-subject information acquiring system of a radio type including a capsule endoscope according to a first embodiment of the present invention. In FIG. 1, the intra-subject information acquiring system includes a receiving device 2 having a radio receiving function, and a capsule endoscope 3 which is inserted into an inside of a body of a subject 1 to capture images in a body cavity and transmit data such as an image signal to the receiving device 2. The intra-subject information acquiring system of a radio type further includes a display device 4 which displays images in the body cavity based on the image signal received by the receiving device 2, and a portable recording medium 5 which transfers data between the receiving device 2 and the display device 4. Here, the intra-subject information acquiring system of a radio type is explained as one example of an intra-subject medical system in which a diagnosis on a subject is made based on intra-subject information (images in a body cavity, for example) obtained from a subject such as a patient.

The receiving device 2 includes an antenna unit 2a which has a plurality of receiving antennas A1 to An attached on an outside surface of the subject 1, and a receiving main body unit 2b which performs a processing on a radio signal received via the plurality of receiving antennas A1 to An, the units being detachably connected to each other via a connector and the like. The receiving antennas A1 to An may be arranged on a jacket which can be worn by the subject 1 for example, and the subject 1 by wearing the jacket may put on the receiving antennas A1 to An. In this case, the receiving antennas A1 to An may be detachable with respect to the jacket. Besides, the receiving antennas A1 to An may be configured such that an antenna main body part at a head part of each of the receiving antennas A1 to An is housed in an antenna pad which can be attached to the body of the subject 1.

The display device 4 is for displaying the images in the body cavity captured by the capsule endoscope 3, and has a configuration of a work station or the like for displaying the images based on data obtained by the portable recording medium 5. Specifically, the display device 4 may have a configuration of displaying the images directly on a CRT display, a liquid crystal display or the like, or a configuration of outputting the images to another medium such as a printer.

The portable recording medium 5 is detachable with respect to the receiving main body unit 2b and the display device 4, and has a configuration such that when inserted to be attached to both of them, information can be output or recorded. In the first embodiment, the portable recording medium 5 is inserted to be attached to the receiving main body unit 2b and records data transmitted from the capsule endoscope 3 therein while the capsule endoscope 3 is moving in the body cavity of the subject 1. After the capsule endoscope 3 is discharged from the subject 1, namely, after the imaging of the inside of the subject 1 is completed, the portable recording medium 5 is taken out of the receiving main body unit 2b and is inserted to be attached to the display device 4, and the display device 4 reads out the data recorded in the portable recording medium 5. For example, by using the portable recording medium 5 such as CompactFlash (registered trademark) for the data transfer between the receiving main body unit 2b and the display device 4, the subject 1 can move freely during the imaging of the inside of the body cavity compared to the case where the receiving main body unit 2b and the display device 4 are connected to each other by a wire. Here, though the portable recording medium 5 is used for the data transfer between the receiving main body unit 2b and the display device 4, the present invention is not necessarily limited to this configuration. For example, another recording device built into the receiving main body unit 2b such as a hard disc drive may be used and the receiving main body unit 2b and the display device 4 may be connected with or without a wire for the data transfer therebetween.

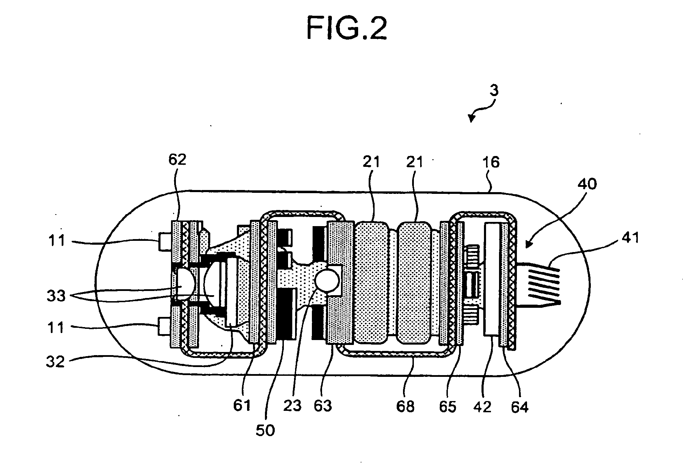

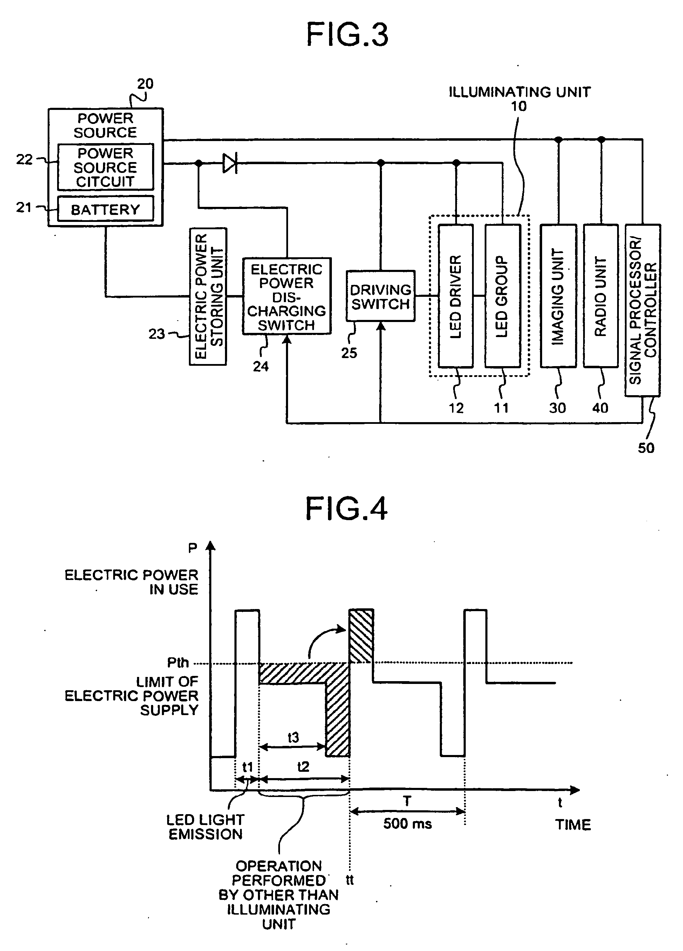

FIG. 2 is a cross sectional view of an internal configuration of the capsule endoscope 3 and FIG. 3 is a block diagram of the configuration of the capsule endoscope 3. FIG. 4 is a time chart showing a state of a power usage of the capsule endoscope 3. The capsule endoscope 3 including an illuminating unit 10 which has a plurality of light emitting diodes (LED) 11 for illuminating the inside of the body cavity of the subject 1 and an LED driver 12 for driving the LED 11; an imaging unit 30 which has a charge coupled device (CCD) 32 for capturing images in the body cavity and an imaging lens 33 for forming images of a subject onto the CCD 32; a radio unit 40 which wirelessly transmits image data captured by the imaging unit 30; a signal processor/controller 50 which performs various signal processings and controls of each unit; and a power source 20 which has a battery 21 for supplying an electric power to each unit, is included in a capsule-shaped casing 16.

The CCD 32 is provided on an imaging substrate 61 and captures images of an area illuminated by an illumination light from the LED 11, and the imaging lens 33 forms images of a subject onto the CCD 32. The LED 11 is mounted on an illuminating substrate 62 and arranged at six points around an optical axis of the imaging lens 33. Besides, the signal processor/controller 50 is mounted on a rear surface side of the imaging substrate 61.

The power source 20 is, for example, constituted by two button batteries 21 whose diameter corresponds to an inner diameter of a body chassis. The batteries 21 can be silver oxide batteries, rechargeable batteries, generating batteries, and the like, for example. The batteries 21 are sandwiched by a power source circuit substrate 63 and a radio substrate 65, and an electric power storing unit 23 which temporarily stores a redundancy of the electric power of the power source circuit 22 and the batteries 21 is mounted on the power source circuit substrate 63 provided at a positive pole side of the batteries 21.

The radio unit 40 is formed by providing a radio electric power amplifier 42 such as an RF transmitter on radio substrates 64 and 65, and an antenna 41 on an outer side of the radio substrates 64. The illuminating substrate 62, the imaging substrate 61, the power source circuit substrate 63, and the radio substrates 64 and 65 are electrically connected suitably via a flexible substrate 68, and sequentially folded to be arranged in the capsule-shaped casing 16.

The capsule-shaped casing 16 has a transparent head cover chassis which has a semispherical shape and covers the illuminating unit 10 and the imaging unit 30, and the body chassis which, having a cylindrical shape, is in engagement with the head cover chassis and kept in a watertight condition, the body chassis being formed by a colored material through which a visual light cannot be transmitted and formed in a size which is small enough to be swallowed from a mouth of the subject 1. The other side where the radio unit 40 is provided of the capsule-shaped casing 16 is connected to the body chassis and has a semispherical shape to cover the antenna 41 therein.

Here, the first embodiment is characterized in that the electric power storing unit 23 shown in FIG. 3 is provided. As shown in FIG. 4, the capsule endoscope 3 performs a processing of repeating, with a predetermined intervening time, to capture an image by using the CCD 32 after the light emission of the LED (a period t1), a signal processing on the captured image, and a processing of wirelessly transmitting the signal-processed data (a period t3). This repetition cycle period is 500 ms, for example.

As shown in FIG. 4, while the light emission by the LED 11 requires an electric quantity over an electric power supply limit Pth, an operation performed by other than the illuminating unit 10 (a period t2) only requires an electric quantity not more than the electric power supply limit Pth. The LED light emission requiring the electric power over the electric power supply limit Pth is operated by a total electric power of the electric power supplied from the power source 20 and the additional electric power stored by the electric power storing unit 23. The electric power supply limit Pth is a threshold value for the electric quantity arbitrarily set at the time of a setting of the capsule endoscope 3, for example.

The electric power storing unit 23 is, for example, realized by a capacitor and the like, and constantly stores, at a constant voltage for the illuminating unit 10, a redundancy of the electric power which is, within the electric power supply limit Pth, supplied from the batteries 21 or the power source circuit 22. This electric power storage is performed within the electric power supply limit Pth. Then, when an electric power discharging switch 24 and a driving switch 25 are switched from OFF state to ON state according to an instruction from the signal processor/controller 50 at the time of the LED light emission, the electric power temporarily stored in the electric power storing unit 23, namely, an electric charge is discharged and supplied to the illuminating unit 10. On this occasion, an electric power corresponding to the electric power supply limit Pth is supplied also from the power source circuit 22 to the illuminating unit 10. Thus, a total electric power of the electric power corresponding to the electric power supply limit Pth and the additional electric power supplied from the electric power storing unit 23 becomes available in the LED light emission. After this LED light emission, an operation can be performed based on the quantity of the electric power within the electric power supply limit Pth which can be fed by the power source circuit 22, and a redundant electric power is stored in the electric power storing unit 23. Here, the driving switch 25 drives the LED driver 12, and the LED driver 12 turns on an electricity to drive each LED 11. The driving switch 25 and the electric power discharging switch 24 become ON state only in the LED light emission (the period t1), and stay OFF state in other points of time.

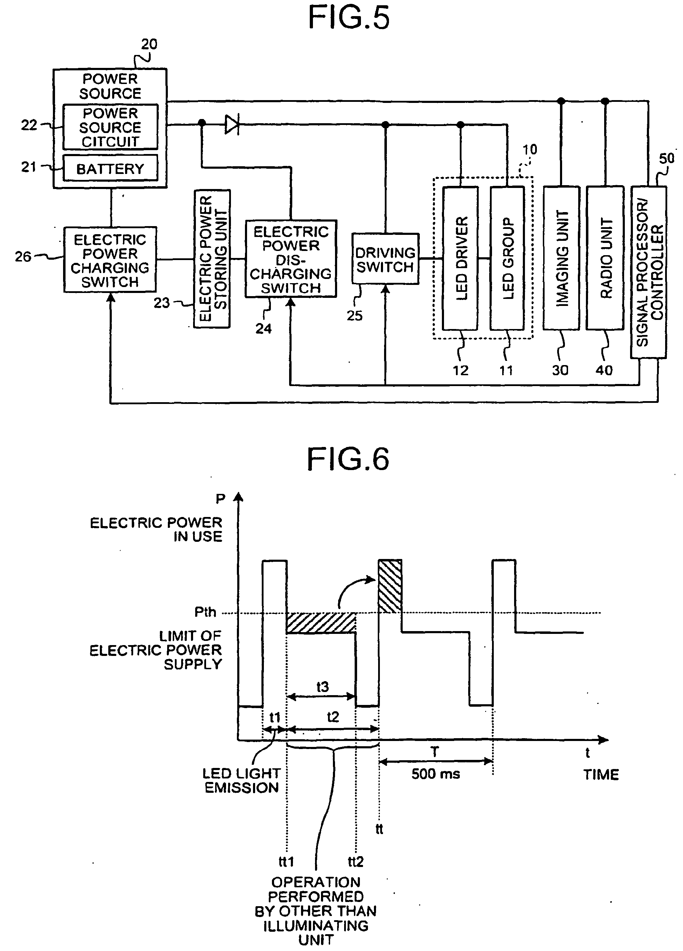

Besides, an electric power charging switch 26 which controls the electric power storage into the electric power storing unit 23 may further be provided as shown in FIG. 5. A switching control of this electric power charging switch 26 is performed by the signal processor/controller 50, and the electric power storing unit 23 performs the electric charge when the electric power charging switch 26 is in ON state and the electric power storing unit 23 does not perform the electric charge when the electric power charging switch 26 is in OFF state. For example, when the electric charge is performed during a period from a time point tt1 when the LED light emission is completed to a time point tt2 when the image data transfer is completed, the electric power charging switch 26 stays ON state during the period from the time point tt1 to the time point tt2, as shown in FIG. 6. In other words, since the electric charge is configured not to be performed in the LED light emission, the electric power corresponding to the electric power supply limit Pth can be supplied from the power source circuit 22 in the LED light emission and thereby the total electric power of the electric power supplied from the power source circuit 22 and the electric power supplied from the electric power storing unit 23 can be made maximum.

In the first embodiment, the electric power storing unit 23 stores a redundant electric power within the electric power supply limit Pth fed by the power source 20, and when an operation such as the operation for the LED light emission which requires an electric power over the electric power supply limit Pth is performed, the stored redundant electric power is added to the electric power for the electric power supply limit Pth to enable such an operation involving the electric power over the electric power supply limit Pth. As a result, the power capacity of the power source can be made small. Specifically, the capacity or the number of the batteries 21 can be reduced, which enables increasing a volume available for function executing units in the capsule endoscope 3 or promoting downsizing the capsule.

### Second embodiment

Next, a second embodiment of the present invention will be explained. In the first embodiment described above, the total electric power of the electric power supplied from the power source circuit 22 and the electric power supplied from the electric power storing unit 23 is equally distributed to each LED 11. However, in the second embodiment, the electric power is distributed separately to an LED 11 which performs a light emission by using the electric power supplied from the power source circuit 22 and to another LED 11 which performs a light emission by using the electric power supplied from the electric power storing unit 23.

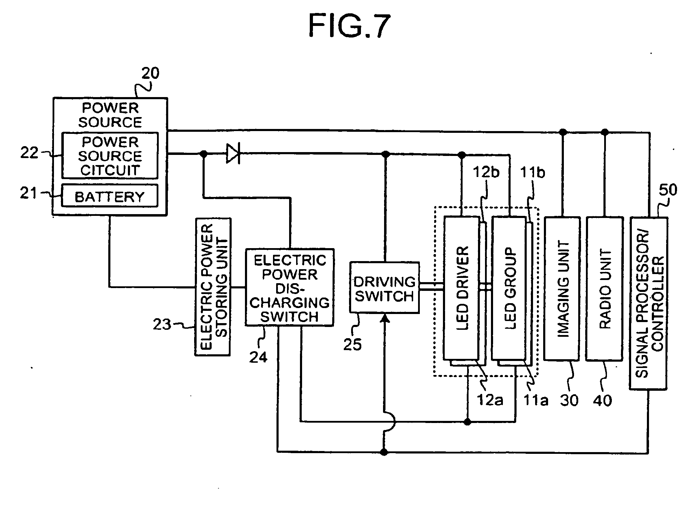

In other words, as shown in FIG. 7, the second embodiment is configured such that a plurality of LEDs 11 are electrically separated into an LED group 11a and into another LED group 11b according to a ratio between the electric power supplied from the power source circuit 22 and the electric power supplied from the electric power storing unit 23, and corresponding LED drivers 12 are electrically separated into an LED driver 12a and into another LED driver 12b, respectively. Specifically, the electric power from the power source circuit 22 is supplied with respect to the LED driver 12a and the LED group 11a, and the electric power stored in the electric power storing unit 23 is supplied with respect to the LED driver 12b and the LED group 11b via the electric power discharging switch 24.

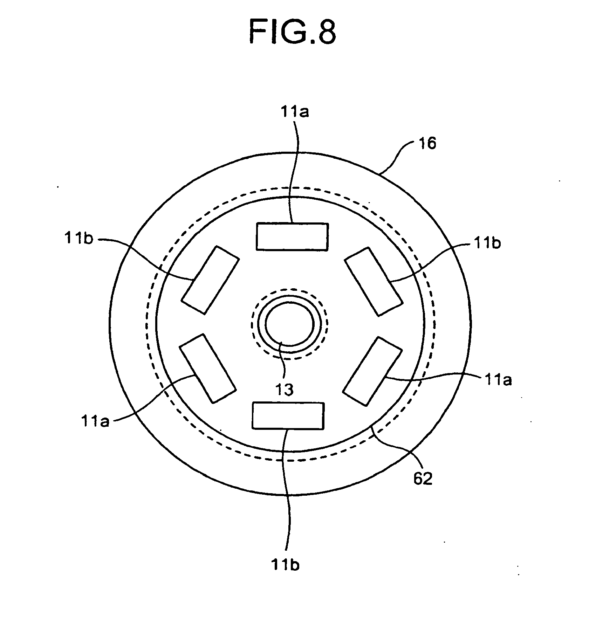

FIG. 8 is a view showing a specific arrangement of the LED 11 and an example of dividing an electric power for each LED 11, the view being seen from a forefront side where the LED 11 is arranged of the capsule endoscope 3. In FIG. 8, six LEDs are provided, and the LED 11a which performs a light emission by using the electric power supplied from the power source circuit 22 and the LED 11b which performs a light emission by using the electric power supplied from the electric power storing unit 23 are alternately arranged in a circle. With such an arrangement, even when a difference arises between an amount of the light emission by the LED 11a and an amount of the light emission by the LED 11b, it is possible to achieve a spatially even light emission. Besides, the LED 11a and the LED 11b may be separately localized in the arrangement. In other words, it is only necessary that a ratio between the number of the LED 11a and the number of the LED 11b is determined depending on the ratio between the electric power supplied from the power source circuit 22 and the electric power supplied from the electric power storing unit 23, and an electric connection is performed so that a source of the electric power for the LED 11a and a source of the electric power for the LED 11b are different to each other.

Though the first and the second embodiments are explained on the assumption that the plurality of LEDs 11 perform the light emission at all times, the present invention is not limited to this and the number of LEDs 11 to emit a light may vary depending on a required amount of light to be emitted. For example, when this is applied to the LED arrangement shown in FIG. 8, the LED 11a is made to emit a light in a normal light emission and the LED 11b is made to emit a light together with the LED 11a at the same time in a case of requiring a large light emission amount. In this case, the electric power discharging switch 24 becomes ON state. By this flexible electric power control, a total usage amount of electric power can be suppressed.

Besides, though the electric power temporarily stored in the electric power storing unit 23 is additionally supplied to a high-power function executing unit (the illuminating unit 10, for example) which requires an electric quantity over the electric power supply limit Pth in the first and the second embodiments described above, the present invention is not limited to this and the stored electric power in the electric power storing unit 23 may be supplied, by the electric power discharging switch 24, to the high-power function executing unit as a function executing unit which, among function executing units in the capsule endoscope, consumes an electric power not less than an average value of the electric power usage (an average electric power value) in the capsule endoscope. In this case, the high-power function executing unit may be any one of the illuminating unit 10, the imaging unit 30, the radio unit 40, and the signal processor/controller 50 of the capsule endoscope.

Furthermore, though the electric power temporarily stored in the electric power storing unit 23 is additionally supplied to the illuminating unit 10 which is one example of the high-power function executing unit requiring an electric quantity over the electric power supply limit Pth in the first and the second embodiments described above, the present invention is not limited to this and may be configured such that the signal processor/controller 50 is provided with a compressor which performs a compression processing on data of images captured by the imaging unit 30 and the electric power discharging switch 24 causes the stored electric power in the electric power storing unit 23 to be additionally supplied to the compressor. Or otherwise, the present invention may be configured such that the signal processor/controller 50 is provided with a signal processing circuit such as an analogue front-end circuit (AFE circuit) which transforms analogue image data captured by the imaging unit 30 into digital image data and the electric power discharging switch 24 causes the stored electric power in the electric power storing unit 23 to be additionally supplied to the signal processing circuit. Namely, the high-power function executing unit described above may be any one of the compressor and the signal processing circuit.

### INDUSTRIAL APPLICABILITY

As described, the body-insertable apparatus according to the present invention is useful for obtaining intra-subject information such as images in the body cavity of the subject, and specifically suitable as a body-insertable apparatus which can instantly obtain a high power with a small electric source capacity and promote downsizing the apparatus at the same time.

## Claims

1. A body-insertable apparatus (3) comprising:
a plurality of function executing units (10, 30, 40, 50) each of which executes a predetermined function;
a power source (20);
an electric power storing unit (23) that is connected to the power source (20) and temporarily stores a redundancy of the electric power supplied from the power source (20); and
an electric power discharging switch (24) that supplies the electric power stored during a temporal period by the electric power storing unit (23) to a high-power function executing unit (10, 30, 40, 50) as a function executing unit (10, 30, 40, 50) that operates intermittently among the function executing units (10, 30, 40, 50) and temporarily consumes a large electric quantity compared to the other function executing units (10, 30, 40, 50), **characterized in that** the power source (20) supplies an electric power to each of the function executing units (10, 30, 40, 50)
and **in that** the high-power function executing unit (10, 30, 40, 50) consumes the electric power supplied from the electric power storing unit (23) together with the electric power directly supplied from the power source (20).

2. The body-insertable apparatus (3) according to claim 1, wherein
the high-power function executing unit (10, 30, 40, 50) includes a driving switch (25) that operates intermittently, and
the electric power discharging switch (24) is turned on/off in synchronization with turning on/off of the driving switch (25).

3. The body-insertable apparatus (3) according to claim 1 or 2, further comprising an electric power charging switch (26) that is provided between the power source (20) and the electric power storing unit (23), and switches on/off a power supply from the power source (20) to the electric power storing unit (23).

4. The body-insertable apparatus (3) according to claim 1, wherein the plurality of function executing units(10, 30, 40, 50) include
an illuminating unit (10) that illuminates an imaging target in imaging;
an imaging unit (30) that captures an image of the imaging target illuminated by the illuminating unit (10);
a radio communication unit (40) that wirelessly transmits the image captured by the imaging unit (30); and
a control unit (50) that controls the illuminating unit (10), the imaging unit (30), and the radio communication unit (40).

5. The body-insertable apparatus (39) according to claim 4, wherein the high-power function executing unit (10, 30, 40, 50) is the illuminating unit (10) that illuminates the imaging target in imaging.

6. The body-insertable apparatus (3) according to claim 4 or 5, wherein the high-power function executing unit (10, 30, 40, 50) is a compressing unit that performs a compression processing on the image captured by the imaging unit (30).

7. The body-insertable apparatus (3) according to claim 4, wherein the high-power function executing unit (10, 30, 40, 50) i s a signal processing circuit that transforms analogue image data captured by the imaging unit (30) into digital image data.

8. The body-insertable apparatus (3) according to claim 5, wherein the illuminating unit (10) includes a plurality of LEDs (11) including an LED (11a) for emitting a light based on the electric power directly supplied from the power source (20) and an LED (11b) for emitting a light based on the electric power supplied from the electric power storing unit (23).

9. The body-insertable apparatus (3) according to claim 1, wherein the electric power storing unit (23) is a capacitor.

10. The body-insertable apparatus according to claim 1, wherein the power source (20) is a battery (21).

11. A body-insertable apparatus (3) according to claim 1, wherein the high-power function executing unit (10, 30, 40, 50) is a function executing unit that, among the function executing units (10, 30, 40, 50), consumes an electric quantity exceeding a threshold value arbitrarily set.

12. A body-insertable apparatus (3) according to claim 1, wherein the
high-power function executing unit (10, 30, 40, 50) is a function executing unit (10, 30, 40, 50) that, among the function executing units (10, 30, 40, 50), consumes an electric quantity not less than an average electric power value.

## Patentansprüche

1. In den Körper einführbares Gerät (3), aufweisend:
eine Mehrzahl eine Funktion ausführende Einheiten (10, 30, 40, 50), von denen jede eine vorgegebene Funktion ausführt;
eine Leistungsquelle (20);
eine elektrische Leistungsspeichereinheit (23), die mit der Leistungsquelle (20) verbunden ist, und vorübergehend ungenutzte von der Leistungsquelle (20) gelieferte Leistung speichert; und
einen elektrischen Leistungsentladungsschalter (24), der die während einer zeitlichen Periode von der elektrischen Leistungsspeichereinheit (23) gespeicherte elektrische Leistung an eine eine Funktion ausführende Hochleistungseinheit (10, 30, 40, 50) als eine eine Funktion ausführende Einheit (10, 30, 40, 50) liefert, die innerhalb der eine Funktion ausführenden Einheiten (10, 30, 40, 50) intermittierend arbeitet und vorübergehend im Vergleich zu den anderen eine Funktion ausführenden Einheiten (10, 30, 40, 50) eine hohe elektrische Leistung aufnimmt,
**dadurch gekennzeichnet, dass** die Leistungsquelle (20) eine elektrische Leistung an jede eine Funktion ausführenden Einheit (10, 20, 30, 40, 50) liefert, und **dadurch**, dass die eine Funktion ausführende Hochleistungseinheit (10, 30, 40, 50) die von der elektrischen Leistungsspeichereinheit (23) gelieferte Leistung zusammen mit der von der Leistungsquelle (20) direkt gelieferten Leistung verbraucht.

2. In den Körper einführbares Gerät (3) nach Anspruch 1, bei dem die eine Funktion ausführende Hochleistungseinheit (10, 30, 40, 50) einen Treiberschalter (25) enthält, der intermittierend arbeitet, und der elektrische Leistungsentladungsschalter (24) synchron mit dem Ein/Aus des Treiberschalters (25) ein-/ausgeschaltet wird.

3. In den Körper einführbares Gerät (3) nach Anspruch 1 oder 2, das ferner einen elektrischen Leistungsladungsschalter (26) aufweist, der zwischen der Leistungsquelle (20) und der elektrischen Leistungsspeichereinheit (23) vorgesehen ist und die Leistungslieferung von der Leistungsquelle (20) zur elektrischen Leistungsspeichereinheit (23) ein-/ausschaltet.

4. In den Körper einführbares Gerät (3) nach Anspruch 1, bei dem die Mehrzahl eine Funktion ausführende Hochleistungseinheiten (10, 30, 40, 50) enthält:
eine Beleuchtungseinheit (10), die bei der Abbildung ein Abbildungsziel beleuchtet;
eine Abbildungseinheit (30), die ein Bild des von der Beleuchtungseinheit (10) beleuchteten Abbildungsziels aufnimmt;
eine Funkkommunikationseinheit (40), die das von der Abbildungseinheit (30) aufgenommen Bild drahtlos überträgt; und
eine Steuereinheit (50), die die Beleuchtungseinheit (10), die Abbildungseinheit (30) und die Funkkommunikationseinheit (40) steuert.

5. In den Körper einführbares Gerät (3) nach Anspruch 4, bei dem die eine Funktion ausführende Hochleistungseinheit (10, 30, 40, 50) die Beleuchtungseinheit (10) ist, die bei der Abbildung das Abbildungsziel beleuchtet.

6. In den Körper einführbares Gerät (3) nach Anspruch 4 oder 5, bei dem die eine Funktion ausführende Hochleistungseinheit (10, 30, 40, 50) eine Komprimierungseinheit ist, die das mit der Abbildungseinheit (30) aufgenommene Bild einer Komprimierungsverarbeitung unterzieht.

7. In den Körper einführbares Gerät (3) nach Anspruch 4, bei dem die eine Funktion ausführende Hochleistungseinheit (10, 30, 40, 50) eine Signalverarbeitungsschaltung ist, die von der Abbildungseinheit (30) aufgenommene analoge Bilddaten in digitale Bilddaten wandelt.

8. In den Körper einführbares Gerät (3) nach Anspruch 5, bei dem die Beleuchtungseinheit (10) eine Mehrzahl LEDs (11) enthält, mit einer LED (11a) zum Emittieren von Licht auf Basis der von der Leistungsquelle (20) direkt gelieferten elektrischen Leistung und einer LED (11b) zum Emittieren von Licht auf Basis der von der elektrischen Leistungsspeichereinheit (23) gelieferten elektrischen Leistung.

9. In den Körper einführbares Gerät (3) nach Anspruch 1, bei dem die elektrische Leistungsspeichereinheit (23) ein Kondensator ist.

10. In den Körper einführbares Gerät nach Anspruch 1, bei dem die Leistungsquelle (20) eine Batterie (21) ist.

11. In den Körper einführbares Gerät (3) nach Anspruch 1, bei dem die eine Funktion ausführende Hochleistungseinheit (10, 30, 40, 50) eine eine Funktion ausführende Einheit innerhalb der eine Funktion ausführenden Einheiten (10, 30, 40, 50) ist, die eine elektrische Leistung aufnimmt, deren Größe einen beliebig eingestellten Schwellenwert überschreitet.

12. In den Körper einführbares Gerät (3) nach Anspruch 1, bei dem die eine Funktion ausführende Hochleistungseinheit (10, 30, 40, 50) eine eine Funktion ausführende Einheit innerhalb der eine Funktion ausführenden Einheiten (10, 30, 40, 50) ist, die eine elektrische Leistung verbraucht, deren Größe einen Durchschnittswert der elektrischen Leistung nicht unterschreitet.

## Revendications

1. Appareil insérable dans un corps (3) comprenant :
une pluralité d'unités d'exécution de fonction (10, 30, 40, 50), chacune desquelles exécute une fonction prédéterminée ;
une source d'énergie (20) ;
une unité de stockage d'énergie électrique (23) qui est connectée à la source d'énergie (20) et stocke temporairement une redondance de l'énergie électrique fournie par la source d'énergie (20) ; et
un commutateur de décharge d'énergie électrique (24) qui fournit l'énergie électrique stockée pendant une période temporelle par l'unité de stockage d'énergie électrique (23) à une unité d'exécution de fonction (10, 30, 40, 50) haute puissance comme une unité d'exécution de fonction (10, 30, 40, 50) qui fonctionne de manière intermittente parmi les unités d'exécution de fonction (10, 30, 40, 50) et consomme temporairement une grande quantité électrique par rapport aux autres unités d'exécution de fonction (10, 30, 40, 50), **caractérisé en ce que** la source d'énergie (20) fournit une énergie électrique à chacune des unités d'exécution de fonction (10, 30, 40, 50), et **en ce que**
l'unité d'exécution de fonction (10, 30, 40, 50) haute puissance consomme l'énergie électrique fournie par l'unité de stockage d'énergie électrique (23) en même temps que l'énergie électrique fournie directement par la source d'énergie (20).

2. Appareil insérable dans un corps (3) selon la revendication 1, dans lequel
l'unité d'exécution de fonction (10, 30, 40, 50) haute puissance inclut un commutateur d'entraînement (25) qui fonctionne de manière intermittente, et
le commutateur de décharge d'énergie électrique (24) est activé/désactivé en synchronisation avec l'activation/désactivation du commutateur d'entraînement (25).

3. Appareil insérable dans un corps (3) selon la revendication 1 ou 2, comprenant en outre un commutateur de charge d'énergie électrique (26) qui est prévu entre la source d'énergie (20) et l'unité de stockage d'énergie électrique (23), et active/désactive une alimentation en énergie de la source d'énergie (20) vers l'unité de stockage d'énergie électrique (23).

4. Appareil insérable dans un corps (3) selon la revendication 1, dans lequel la pluralité d'unités d'exécution de fonction (10, 30, 40, 50) incluent
une unité d'éclairage (10) qui éclaire une cible d'imagerie pendant une imagerie ;
une unité d'imagerie (30) qui capture une image de la cible d'imagerie éclairée par l'unité d'éclairage (10) ;
une unité de communication radio (40) qui transmet sans fil l'image capturée par l'unité d'imagerie (30) ; et
une unité de commande (50) qui commande l'unité d'éclairage (10), l'unité d'imagerie (30) et l'unité de communication radio (40).

5. Appareil insérable dans un corps (3) selon la revendication 4, dans lequel l'unité d'exécution de fonction (10, 30, 40, 50) haute puissance est l'unité d'éclairage (10) qui éclaire la cible d'imagerie pendant l'imagerie.

6. Appareil insérable dans un corps (3) selon la revendication 4 ou 5, dans lequel l'unité d'exécution de fonction (10, 30, 40, 50) haute puissance est une unité de compression qui effectue un traitement de compression sur l'image capturée par l'unité d'imagerie (30).

7. Appareil insérable dans un corps (3) selon la revendication 4, dans lequel l'unité d'exécution de fonction (10, 30, 40, 50) haute puissance est un circuit de traitement de signaux qui transforme des données d'image analogiques capturées par l'unité d'imagerie (30) en données d'image numériques.

8. Appareil insérable dans un corps (3) selon la revendication 5, dans lequel l'unité d'éclairage (10) inclut une pluralité de LED (11) incluant une LED (11a) pour émettre une lumière sur la base de l'énergie électrique fournie directement de la source d'énergie (20) et une LED (11b) pour émettre une lumière sur la base de l'énergie électrique fournie par l'unité de stockage d'énergie électrique (23).

9. Appareil insérable dans un corps (3) selon la revendication 1, dans lequel l'unité de stockage d'énergie électrique (23) est un condensateur.

10. Appareil insérable dans un corps (3) selon la revendication 1, dans lequel la source d'énergie (20) est une batterie (21).

11. Appareil insérable dans un corps (3) selon la revendication 1, dans lequel l'unité d'exécution de fonction (10, 30, 40, 50) haute puissance est une unité d'exécution de fonction qui, parmi les unités d'exécution de fonction (10, 30, 40, 50), consomme une quantité électrique excédant une valeur de seuil fixée arbitrairement.

12. Appareil insérable dans un corps (3) selon la revendication 1, dans lequel
l'unité d'exécution de fonction (10, 30, 40, 50) haute puissance est une unité d'exécution de fonction (10, 30, 40, 50) qui, parmi les unités d'exécution de fonction (10, 30, 40, 50), consomme une quantité électrique non inférieure à une valeur moyenne d'énergie électrique.
